(19)

Europäisches Patentamt
European Patent Office
Office européen des brevets

(11)  **EP 1 840 557 A1**

(12)  **EUROPEAN PATENT APPLICATION**

(43) Date of publication:
**03.10.2007 Bulletin 2007/40**

(51) Int Cl.:
*G01N 21/35* (2006.01)    *G01N 33/14* (2006.01)

(21) Application number: **06111827.9**

(22) Date of filing: **28.03.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **FOSS Analytical A/S
3400 Hilleroed (DK)**

(72) Inventor: **Hjul, Henrik Vilstrup
4000, Roskilde (DK)**

(54)  **Determination of Components of Liquids**

(57)  An apparatus for detecting a component of a liquid sample (12) comprises measurement instrumentation (4) adapted to detect the component by one or both of a quantitative and a qualitative measurement of absorption of optical radiation by gas from a headspace (14) above the liquid (12) and a gas flow system (6) configured to remove gas from and to recirculate extracted gas to the headspace (14) through the liquid sample (12). A dosing device (8) may also be provided for transfer of a reagent from a reservoir (32) into the liquid (12) to effect release into the liquid of a species indicative of the component of interest for extraction into the headspace (14) by the recirculated gas.

**Fig. 1**

EP 1 840 557 A1

**Description**

The present invention relates to an apparatus for the quantitative and/or the qualitative determination of components of liquids and in particular to their determination by means of optical spectroscopy.

In the context of the present invention "optical" shall be taken to mean any or all of that part of the electromagnetic spectrum extending from and including ultraviolet radiation to and including far infra-red radiation. Related terms shall be construed accordingly.

It is well known that the presence and/or in particular the amount of sulphur dioxide ($SO_2$) and other components of interest in a beverage product or an intermediate product of the beverage production process may be determined by the optical analysis of headspace gases above a liquid sample. By measuring the presence in the gas of species indicative of the one or more component of interest in the liquid (perhaps being one and the same) then the presence and/or particularly the amount of that component can be readily determined.

It is known, from for example US 5 426 593 of Seiden et al, to determine one or more of the oxygen ($O_2$); carbon dioxide ($CO_2$); and nitrogen ($N_2$) content of a so-called "beverage gas" present in the headspace of a sealed, end-consumer container (here a metal can). The liquid beverage is firstly degassed using ultrasound, the can is then pierced and a sample of the headspace beverage gas removed for optical analysis in order to identify the presence of the one or more components of interest in the liquid by measuring their presence in the headspace beverage gas.

It is further known, from for example EP 1 308 713 of Qmet ApS, to determine components of a liquid sample by optical analysis of a headspace gas that is allowed to become established in a sealed sample container having a volume greater than that of the liquid sample. This document discloses in particular a method for the analysis of $SO_2$ in wine or other beverage by use of infra-red measurement instrumentation.
According to this method a liquid sample of a specific volumetric size is introduced into a sealed container having a fluid holding volume in excess of the specific volumetric size. Carbon dioxide ($CO_2$) and other gases that may interfere with the measurement are first removed from the headspace gases then the pH of the liquid is adjusted in order to free $SO_2$ present in the liquid. Thereafter a concentration of $SO_2$ is allowed to establish in the gaseous headspace. A gas sample is then removed from the headspace and the concentration of $SO_2$ in that gas sample is measured by means of the infra-red measurement instrumentation adapted to measure the attenuation of infra-red radiation transmitted through the sample. From this measurement the content of $SO_2$ in the beverage is determined.

It is desirable to enhance the extraction from the liquid sample of species indicative of the one or more components of interest into the headspace and so speed up the analysis. It is a generally known technique to bubble a carrier gas, for example nitrogen, through the liquid for the order of fifteen to twenty minutes to achieve this. However this has a problem that the now trace amounts of the species are difficult to detect accurately in the presence of a relatively large volume of the carrier gas which itself may have a small but significant spectral contribution to the optical wavelength regions employed for the determination.

According to the present invention there is provided an apparatus as described in and characterised by the present Claim 1. By arranging a gas flow system in operable connection with a sample container to provide an external gas flow path coupling headspace gas to a liquid holding volume of the container then headspace gas may be passed through the liquid at least once to aid extraction of a species indicative of the a component of interest into the headspace. In this manner a rapid extraction from the liquid sample and an increased concentration of species in the headspace gas is achieved without dilution by a carrier gas.

Preferably the optical measurement instrumentation is disposed in gaseous communication, usefully in-line, with the gas flow system so that headspace gas enriched with a species indicative of a component of interest after having passed through the liquid sample at least once can be provided simply to the optical instrumentation for measurement without stopping the flow of gas in the gas flow system.

These and other advantages will become apparent from a reading of the following description of an exemplary embodiment of the apparatus according to the present invention made with reference to the drawings of the accompanying figures, where:

Fig. 1 shows schematically an embodiment of the present invention configured for $SO_2$ determination in a beverage;

Fig. 2 illustrates the evolution over time of absorption spectra generated by the embodiment of Fig.1;

Fig. 3 illustrates the change in absorption over time of the $SO_2$ related absorption band of Fig. 2;

Fig. 4 illustrates the change in absorption of the band of Fig. 3 corrected for a contribution by ethanol; and

Fig. 5 illustrates a standardisation curve used to establish a relationship between measured absorption and $SO_2$ content.

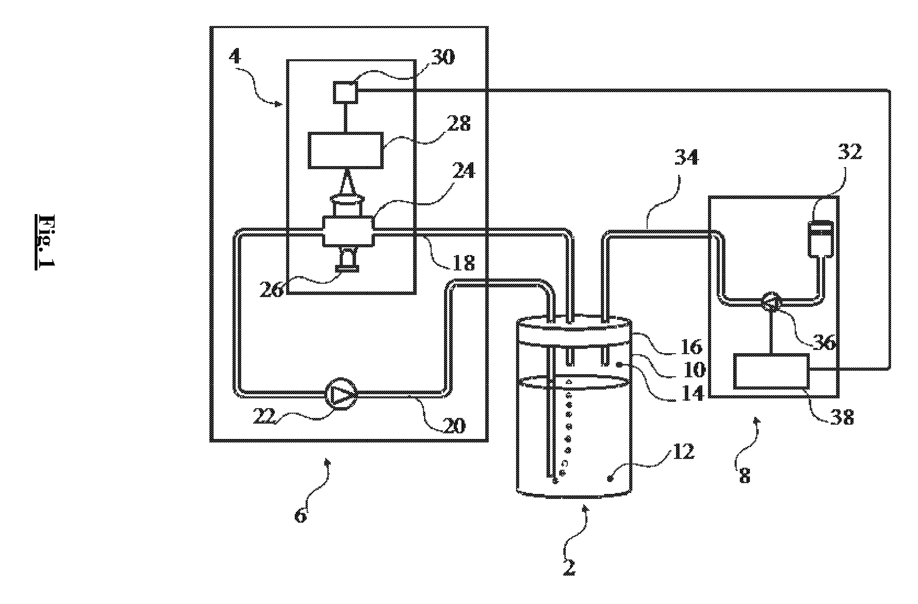

Considering now the exemplary apparatus which is illustrated schematically in Fig. 1. Although this will be described in connection with the determination of an amount of $SO_2$ in a beverage, such as wine, cider, beer or fruit juice, or an intermediate product of the beverage production process it is not intended that the present invention is limited only to this application.

As illustrated, the apparatus of Fig. 1 can be considered to comprise four functional elements: a sample holder 2; optical measurement instrumentation 4; a gas flow system 6; and dosing apparatus 8.

The sample holder 2 of the present embodiment comprises a liquid container 10 which is dimensioned to define an inner volume greater than an expected predetermined volume of a liquid sample (wine say) 12 which will, in use, be transferred into and retained by the container 10. This permits the formation of a gas containing headspace volume 14 in the container 10 above the liquid 12. The sample holder 2 additionally here comprises a preferably removable closure 16 to seal the container 10 against unintentional egress of fluid, in particular gas. According to alternative embodiments the sample holder 2 may not be an element of an apparatus according to the present invention and may, for example, comprise a sealed end user container, such as a corked bottle, a can or waxed paper container, and appropriate fluid connections established by piercing the container or the sample holder 2 may include a single-use container.

The gas flow system 6 is provided in gas communication with the inner volume of the container 10, here via the closure 16 such that gas from the headspace volume 14 may be extracted from and returned to the container 10. The gas flow system 6 of the present embodiment comprises an extraction conduit portion 18, coupled to the headspace volume 14, and a return conduit portion 20, coupled to the inner volume of container 10 at a location such that in use gas from the conduit 20 is supplied in to the liquid sample 12. A circulation pump 22 is operably connected to the gas flow circuit 6 to effect recirculation of gas from the head space 14 to the liquid 12. The circulation pump 22 may be considered as delimiting these extraction and return conduit portions 18, 20 which are respectively located upstream and downstream the pump 22.

The optical measurement instrumentation 4 is disposed to receive gas from gas flow system 6 and is provided with a measurement station 24 at which the interaction of optical radiation with the gas is monitored. In the present embodiment the measurement station 24 is defined by a cuvette which is connected in-line to the gas flow circuit of the flow system 6. Alternatively the measurement station 24 may, for example, comprise a suitably optically transparent region of the extraction or supply gas conduit portions 18,20. The instrumentation 4 comprises complementary optical radiation supply 26 and detection element 28 cooperable to monitor absorption of optical radiation from the supply 26 by gas from the headspace 14. When configured to measure $SO_2$ which has a known absorption peak in the wavelength region around 1380 cm$^{-1}$ then the optical radiation supply 26 may suitably comprise an infra-red radiation supply, emitting in the appropriate wavelength region. It is also known that for wine and other alcoholic beverages ethanol also contributes to absorption in that region and that ethanol itself has an absorption peak at around 1250 cm$^{-1}$. The optical radiation supply 26 may therefore usefully be configured to generate infra-red radiation containing radiation at these two absorption wavelengths, such as for example generating throughout the wavelength region between 1000 cm$^{-1}$ and 1900 cm$^{-1}$ or alternatively in a narrow band around 1250cm$^{-1}$ and one around 1380cm$^{-1}$.

The detection element 28 may comprise a conventional Fourier Transform Infra-Red (FTIR) spectrometer configured to operate in a known manner and disposed to detect optical radiation from the supply 26 after its interaction with gas in the cuvette 24. In the present embodiment the FTIR spectrometer 28 and the supply 26 are mutually arranged to operate in transmission mode, that is a mode where radiation is transmitted through the cuvette 24 from the supply 26 to be detected by the spectrometer 28.

According to alternative embodiments these supply 26 and detection 28 elements may be configured to operate in known reflectance or transflectance modes. Indeed, the FTIR spectrometer may be substituted for other known detection elements, such as a fixed or a scanning dispersion element monochromator or a detector and filter configuration, suitable for monitoring absorption of optical radiation by a species in the gas (here $SO_2$) which is indicative of a component of interest (here also $SO_2$) in the liquid sample 12 (here wine).

A signal processor 30, here shown as integral with the optical measurement instrumentation 4, is connected to receive an output from the FTIR spectrometer 28 which is representative of wavelength correlated intensities of detected optical radiation and to analyse the received output signal to establish a quantitative and additionally or optionally a qualitative measure of predetermined components of the gas in a manner known in the art.

The signal processor 30 is, in the present embodiment, configured to generate a control signal dependent on this analysis for use within the apparatus and also to analyse the output in a known manner in order to measure an amount in the gas in the cuvette 24 of the species indicative of the component of interest and to determine therefrom the amount of the (here the same) component of interest in the liquid sample 12 (here wine). A human discernable indication of the determined amount is also generated by the signal processor 30.

The dosing apparatus 8 comprises a reagent reservoir 32 which is fluidly connectable to the inner volume of the container 10 via a conduit 34; a dosing pump 36 for effecting the transfer of reagent from the reservoir 32 to the container 10; and a controller 38 for controlling the operation of the pump 36. The controller 38 is connected to the signal processor 30 to receive the control signal and to trigger the operation of the pump 36 in dependence thereof.

In the present embodiment, where the apparatus is configured to monitor $SO_2$ in wine or other beverage, then the reagent is an acid which when added to the wine adjusts the pH of the liquid to cause the release into the wine of the otherwise bound $SO_2$. This freed $SO_2$ is then available to be extracted from the wine and in to the headspace volume 14 by the recirculated headspace gas being passed through the wine sample 12 from the flow system 6. In this manner a gas may be rapidly generated in the headspace 14 that is enriched with a species indicative of a component of interest (here both $SO_2$).

Generally the reagent in the reservoir 32 may be selected to either effect release into the liquid sample of the particular component of interest or to form a gaseous (or at least a volatalisable) species indicative of the presence and/or amount of the component of interest in the liquid sample. In some applications the dosing apparatus 8 may be omitted.

In operation the liquid sample 12 (here wine) of a known volume less than the inner volume defined by the container 10 is introduced into the sample holder 2. According to alternative embodiments the liquid sample 12 may be introduced manually from a larger reservoir (such as a bottled final product or a fermentation vat), for example by means of a syringe or poured from a measurement beaker, or may be introduced automatically in a controlled manner from a tube or pipeline that is connected to a process line for the liquid, using for example using know control valves connected between the pipeline and the container 10. The closure 16 may be provided with suitable access ports to facilitate this and other necessary fluid transfers.

In certain other embodiments where the components of the ambient air may interfere with the optical measurements then an inert gas, such as nitrogen ($N_2$) may be first introduced into the container 10, to be partially displaced by the liquid sample 12 as it is introduced into the sample holder 2. A simple gas relief vent of known construction may be provided in the container 10 or the closure 16 through which the displaced $N_2$ may exit. The gas in the headspace volume 14 will then, in this particular embodiment, initially consist of $N_2$ into which the species indicative of the component of interest will eventually pass.

Once the wine sample 12 is transferred and the container 10 sealed against unwanted transfer of fluids the optical measurement instrumentation 4 may be operated in order to establish a so-called 'base-line' spectral measurement which, in the present exemplary embodiment, is stored for later use and with respect to which other spectral measurements will be established. In this manner any effects of the cuvette 24, the aging of the optical radiation supply 26, etc, on the optical absorption by the predetermined components of the gas may be compensated for in subsequent spectral measurements. The circulation pump 22 is then started and gas is circulated between the headspace 14 volume and the liquid sample 12 volume of the container 10 via the external extraction and supply portions 18, 20 of the gas flow system 6.

During an initial operating period of the circulation pump 22 primarily ethanol and water will evaporate into the headspace volume 10 to be recirculated. The optical measurement instrumentation 4 is operated at least once during this initial operating period to generate a so called 'background' absorption spectrum which, in the present exemplary embodiment, is stored for later use.

Circulation of gas between the headspace volume 14 and the wine 12 during this initial operating period causes an increase with time of the amount of ethanol and water vapour in the headspace gas and it is preferable that the optical measurement instrumentation 4 is operated a plurality of times throughout this initial operating period to generate a plurality of background absorption spectra for comparison in the signal processor 30 in order to determine when substantially all of the ethanol has been extracted into the gas.

Alternatively, the optical measurement instrument 4 may be operated in a timed relationship with the commencement of gas recirculation by the circulation pump 22 to generate a single background absorption spectrum when substantially all of the ethanol has been extracted. According to this alternative embodiment the timing of the generation of this spectrum can readily be established by simple trial and error using a reference sample and may be set by a user or preset in a factory.

It is well known that an absorption peak at around 1250 cm$^{-1}$ is directly associated with the amount of ethanol in the gas. The signal processor 30 is configured to operate during this initial period to compare successive background spectra to determine a condition when substantially all of the ethanol has been extracted. This may be achieved by adapting the signal processor 30 to monitor the intensity of the absorption band directly associated with ethanol and to establish when the variation in monitored intensity falls below a predetermined threshold, indicating this condition has been reached.

Once this condition is reached, or optionally after a predetermined time from starting the circulation pump 22, the operation of the dosing pump 36 is triggered and reagent from reservoir 32 is transferred to the liquid sample 12 whilst the headspace gas continues to be recirculated. In the present embodiment upon determination that substantially all of the ethanol has been extracted the signal processor 30 issues a control signal to the controller 38 of the dosing apparatus 8, triggering the operation of the dosing pump 36. The addition of the acid reagent to the wine sample 12 causes $SO_2$ to be liberated which is then extracted in to the headspace 14 under the influence of the recirculating gas from the gas flow system 6 which is passing through the wine sample 12.

The optical measurement instrumentation 4 is preferably operated a plurality of times during the period that

the $SO_2$ is liberated into the headspace volume 14 to generate so called 'measurement' absorption spectra containing features resulting from the absorption of optical radiation by the $SO_2$. The signal processor 30 analyses the so generated spectra to measure the amount of so liberated $SO_2$ and to determine from this the amount of $SO_2$ in the wine sample 12. In one embodiment the signal processor 30 is programmed to calculate from a measurement spectrum a maximum height of the absorption band at around 1380 $cm^{-1}$ and from this determine the amount of $SO_2$ in the headspace gas. This represents the total amount of $SO_2$ which was present in the wine sample 12. A correlation between the so calculated maximum height and the $SO_2$ can be readily established empirically using samples having known $SO_2$ concentrations. This correlation can then be made available for use within the signal processor 30 in order to determine the amount of $SO_2$ represented by the calculated maximum height.

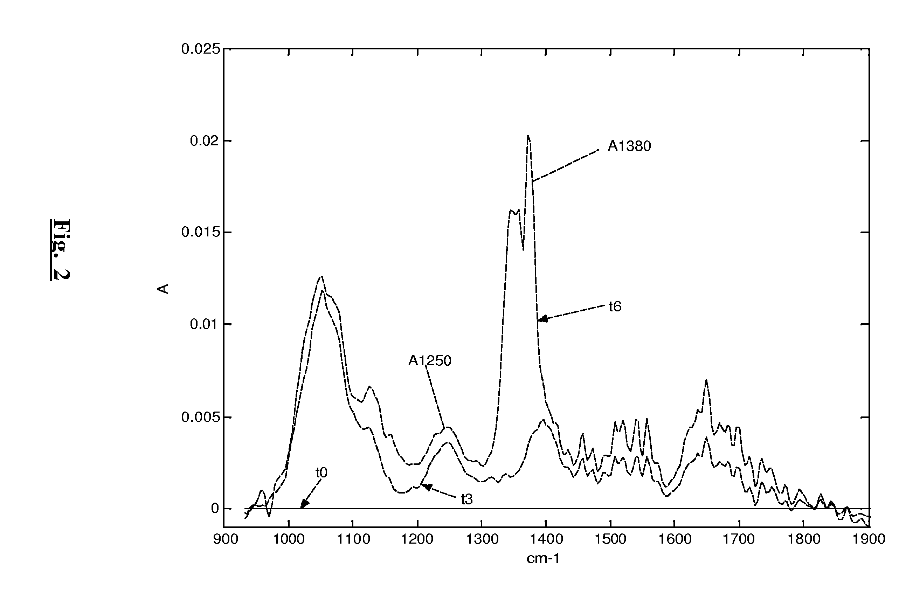

Referring now to Fig. 2, representative absorption spectra generated during the operation of the optical measurement instrumentation 4 are illustrated in a plot of absorption intensity (A) against wavelength in $cm^{-1}$. These represent spectra that have been corrected for spectral artefacts unrelated to the absorption by the gas. This is achieved through a subtraction of the base-line spectrum. Of interest is the absorption peak ($A_{1250}$) at around 1250 $cm^{-1}$ which is related directly to ethanol and the peak ($A_{1380}$) at around 1380 $cm^{-1}$ which is related primarily to $SO_2$. The spectrum designated to in Fig. 2 is the corrected base-line spectrum, as it is corrected by subtraction of itself then the result is the straight line illustrated. The spectrum designated $t_3$ is the corrected background spectrum and that spectrum designated $t_6$ is the corrected measurement spectrum. These spectra illustrate how absorption intensities of these bands evolve with time t (where $t_0 < t_3 < t_6$) and the appearance of the high absorption at 1380 $cm^{-1}$ can be directly associated with the liberation of $SO_2$ after addition of the acid reagent.

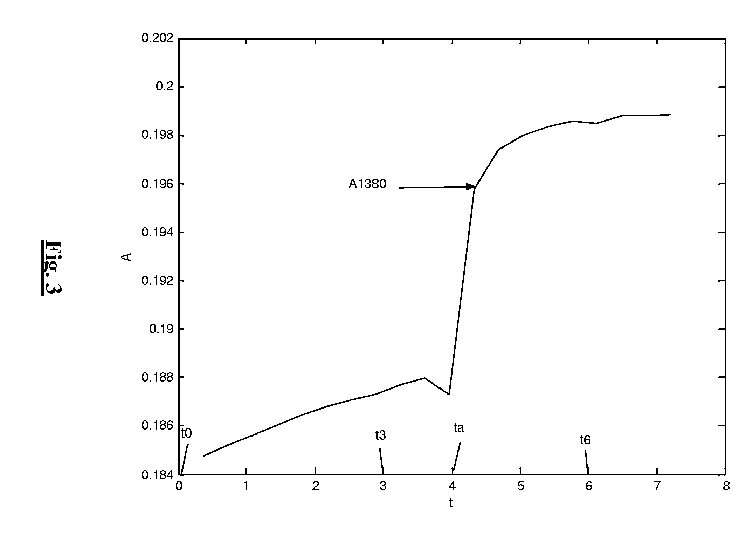

This evolution can be more clearly seen in Fig. 3 which provides an exemplary plot of absorption peak $A_{1380}$ with time (t) over an eight minute operating period of the circulation pump 22. The base-line spectrum $t_0$ is obtained at or before time 0 (switch on of the pump 22 to begin recirculation of headspace gas) and the background spectrum $t_3$ is that obtained 3 minutes later. The reagent is added at time $t_a$ and the absorption peak $A_{1380}$ increases rapidly thereafter until an equilibrium state is reached when substantially no more $SO_2$ is released from the liquid sample 12. At this time, here shown as some six minutes after the commencement of the operation of the circulation pump 22, the measurement spectrum $t_6$ is obtained. It will be appreciated that these mentioned above times are illustrative only and are employed merely to clarify the operation of the apparatus according to the present invention.

The rather unstable (generally increasing) baseline intensity at times earlier than $t_a$ is due to an increasing contribution to the absorption peak $A_{1380}$ by ethanol, which is known also to absorb in this region. However the absorption peak $A_{1250}$ which is illustrated in Fig. 2 is known to be directly related to ethanol. The signal processor 30 can most usefully be additionally configured to determine an amount of ethanol present in the headspace gas from a calculation based on the height of this peak $A_{1250}$ derived from an analysis by the signal processor 30 of the stored background spectrum $t_3$ (illustrated in Fig. 2). The signal processor 30 then calculates an adjusted height $A_{adjusted}$ of the $A_{1380}$ peak corrected for the contribution to it by ethanol by means of a simple formula:

$$A_{adjusted} = A_{1380} - 0.69 A_{1250} \tag{1}$$

(where the coefficient 0.69, is determined from an analysis of the ethanol contribution to the $t_3$ spectrum at 1250 $cm^{-1}$ and at 1380 $cm^{-1}$)

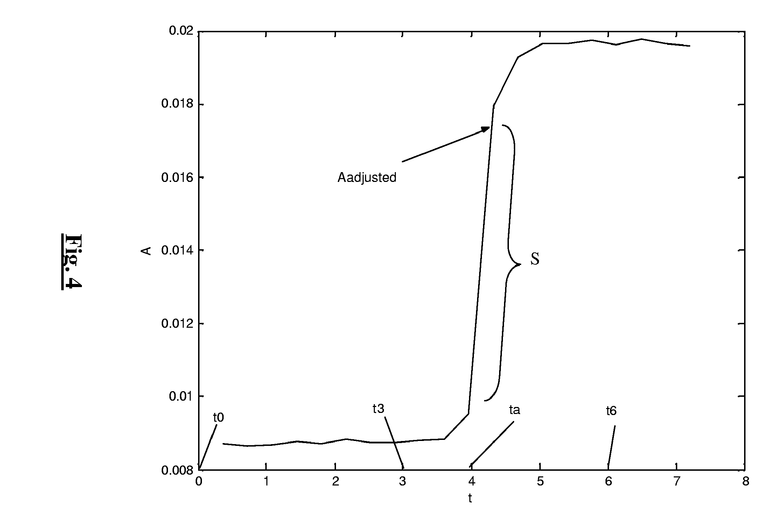

The variation of this adjusted peak height $A_{adjusted}$ with time is illustrated in Fig. 4 where features in common with Fig. 3 are identified using the same reference symbols). The actual concentration of $SO_2$ in the wine sample 12 is now proportional to the two levels before and after the addition of the acid reagent $t_a$.

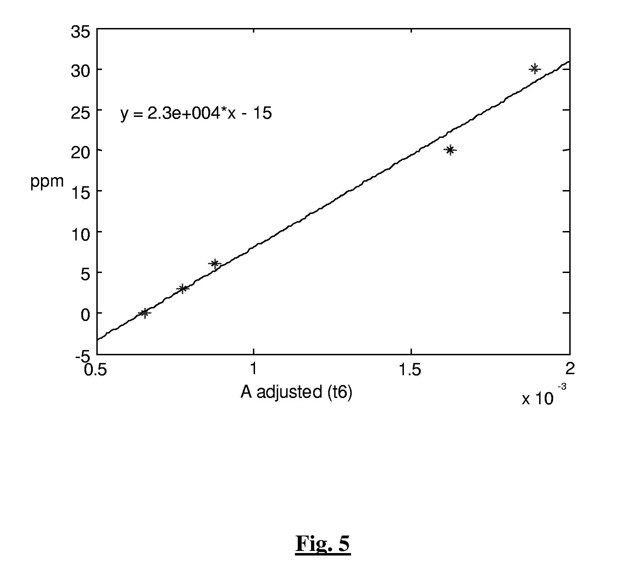

The relationship between the height of the absorption peak due to $SO_2$, $A_{adjusted}$ and the amount of $SO_2$ in the liquid sample 12 can be readily obtained by means of the known standard addition technique. In this technique known amounts of $SO_2$ are added to the liquid sample 12 and with each addition the height of the absorption peak $A_{adjusted}$ is determined. This is illustrated in Fig. 5 which shows an exemplary plot of added $SO_2$ in parts per million (ppm) in the y-axis against the absorption peak height $A_{adjusted}$ in the x-axis, here obtained using the measurement spectrum $t_6$ illustrated in Fig. 2.

The straight line fit yields a relationship for use in the signal processor 30 in order to quantitatively determine the amount of $SO_2$ in the liquid sample 12 from an analysis of the optical absorption by headspace gas. As illustrated for the particular wine type of the liquid sample 12 this relationship may be expressed as:

$$Y = 2.3e^{+4} * X - 15 \tag{2}$$

Additionally or alternatively the amount of $SO_2$ in the wine sample 12 may be determined from the rate of change of the $A_{adjusted}$ peak with time after the addition of the acid reagent at time $t_1$ (illustrated by the slope S of the $A_{adjusted}$ curve in Fig. 4).

As described above it can be readily appreciated that the amount of $SO_2$ in wine may be determined by a comparison of only two absorption bands, those at 1250 cm$^{-1}$ ($A_{1250}$) and 1380 cm$^{-1}$ ($A_{1380}$), known to be associated with ethanol and with $SO_2$ respectively. The optical measurement instrumentation 4 may be suitably configured to measure absorption at just these wavelengths (or a narrow wavelength band about these wavelengths). Such instrumentation 4 may comprise an infra red source 26 selected to emit in a wavelength region including these two absorption bands and a detection element comprising a detector and bandpass (or notch) filter arrangement to selectively expose the detection element to the two absorption bands $A_{1250}$ and $A_{1380}$, and may conveniently comprise only two filters.

## Claims

1. An apparatus for detecting a component of a liquid sample (12) comprising measurement instrumentation (4) adapted to detect the component by one or both of a quantitative and a qualitative measurement of absorption of optical radiation by gas from a headspace (14) above the liquid (12); **characterised in that** the apparatus further comprises a gas flow system (6) configured to extract gas from the headspace (14) and to recirculate extracted gas to the headspace (14) through the liquid sample (12).

2. An apparatus as claimed in Claim 1 **characterised in that** the measurement instrumentation (4) is disposed in gas communication with the gas flow system (6).

3. An apparatus as claimed in Claim 2 **characterised in that** the measurement instrumentation (4) is disposed to measure absorption by gas from the headspace (14) flowing in the gas flow system (6).

4. An apparatus as claimed in Claim 3 **characterised in that** the measurement instrumentation (4) comprises a through flow cuvette (24) located in-line in the gas flow system (6), said cuvette (24) being at least partially composed of a material transparent to optical radiation to thereby define a measurement location.

5. An apparatus as claimed in any preceding claim **characterised in that** there is further provided a reagent dosing apparatus (8) adapted to transfer an amount of reagent from a reservoir (32) into the liquid sample (12) upon receipt of a trigger signal.

6. An apparatus as claimed in Claim 4 **characterised in that** the optical measurement instrumentation (4) is adapted to measure temporal variations in absorption by recirculated gas from the headspace (14) before transfer of the reagent from the reservoir (32) **and in that** the apparatus further comprises means (30) to monitor the measured temporal variations and to provide the trigger signal dependent on a comparison of the monitored variations with a predetermined threshold.

7. An apparatus as claimed in Claim 5 or Claim 6 **characterised in that** the optical measurement instrumentation (4) is adapted to measure the absorption by recirculated headspace gas a predetermined time after the receipt of the trigger signal by the reagent dosing apparatus (8) for use in the determination of an amount of said component in the liquid sample.

8. An apparatus as claimed in any one of the claims 5 to 7
   **characterised in that** the reagent dosing apparatus (8) is an acid dosing apparatus and **in that** the optical measurement instrumentation (4) is adapted to quantitatively measure absorption of infra red radiation at least in a wavelength region susceptible to absorption by sulphur dioxide and **in that** processing means (30) is provided to determine using the quantitative measurement an amount of sulphur dioxide in the liquid.

**Fig. 1**

**Fig. 2**

**Fig. 3**

**Fig. 4**

**Fig. 5**

**European Patent Office**

**EUROPEAN SEARCH REPORT**

Application Number

EP 06 11 1827

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2003/178323 A1 (FIEDLER ANDREAS ET AL) 25 September 2003 (2003-09-25)<br>* abstract; figure 1 *<br>* paragraph [0023] - paragraph [0025] *<br>* paragraph [0052] - paragraph [0053] *<br>* paragraph [0081] - paragraph [0084] *<br>* paragraph [0087] *<br>* paragraph [0089] - paragraph [0090] *<br>* paragraph [0104] - paragraph [0106] *<br>----- | 1-8 | INV.<br>G01N21/35<br>G01N33/14 |
| D,Y | EP 1 308 713 A (QMET APS) 7 May 2003 (2003-05-07)<br>* abstract; figure 1 *<br>* paragraph [0009] - paragraph [0012] *<br>* paragraph [0014] *<br>* paragraph [0017] - paragraph [0021] *<br>----- | 1-8 | |
| A | US 5 614 718 A (BRACE ET AL) 25 March 1997 (1997-03-25)<br>* abstract; claims 1-12; figures 3,8 *<br>----- | 1 | |

TECHNICAL FIELDS SEARCHED (IPC)

G01N

The present search report has been drawn up for all claims

| Place of search | Date of completion of the search | Examiner |
|---|---|---|
| The Hague | 2 August 2006 | Bockstahl, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 840 557 A1**

### ANNEX TO THE EUROPEAN SEARCH REPORT
### ON EUROPEAN PATENT APPLICATION NO.

EP 06 11 1827

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2006

| Patent document cited in search report | | Publication date | Patent family member(s) | | Publication date |
|---|---|---|---|---|---|
| US 2003178323 | A1 | 25-09-2003 | AT | 275725 T | 15-09-2004 |
| | | | AU | 6598901 A | 03-12-2001 |
| | | | DE | 10024947 A1 | 14-02-2002 |
| | | | WO | 0190742 A1 | 29-11-2001 |
| | | | EP | 1285266 A1 | 26-02-2003 |
| | | | ES | 2227218 T3 | 01-04-2005 |
| | | | ZA | 200209436 A | 20-11-2003 |
| EP 1308713 | A | 07-05-2003 | CA | 2465544 A1 | 08-05-2003 |
| | | | WO | 03038411 A1 | 08-05-2003 |
| | | | MX | PA04004124 A | 29-10-2004 |
| | | | US | 2004238745 A1 | 02-12-2004 |
| US 5614718 | A | 25-03-1997 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5426593 A, Seiden
- EP 1308713 A